# EUROPÄISCHE PATENTANMELDUNG

(11) **EP 3 385 366 A1**
(43) Veröffentlichungstag der Anmeldung: **10.10.2018**
(21) Anmeldenummer: 17164767.0
(22) Anmeldetag: 04.04.2017
(51) Int. Cl.: C12M 1/34, G01N 33/487

(54) **VERFAHREN ZUR KONTROLLE EINES BIOTECHNOLOGISCHEN PROZESSES**

(71) Anmelder: Siemens Aktiengesellschaft, 80333 München (DE)
(72) Erfinder: JOKSCH, Martin, 3423 St.Andrä-Wördern (AT)
(74) Vertreter: Maier, Daniel Oliver

(57) **Zusammenfassung**

Die Erfindung bezieht sich auf ein Verfahren zur Kontrolle eines biotechnologischen Prozesses. Bei diesen biotechnologischen Prozessen werden während eines Prozessverlaufs Ausgangsstoffe mit Hilfe von Biomasse in Produkte umgesetzt und es werden wichtige Prozessparameter für eine Überwachung ermittelt. Während eines Verlaufs des biotechnologischen Prozesses wird periodisch wiederkehrend eine aktuelle Konzentration der im Prozess eingesetzten Biomasse abgeschätzt (1). Dann werden periodisch wiederkehrend aktuelle Messwerte von messbaren Prozessparametern bestimmt (2) und daraus aktuelle Werte für zusätzliche Prozessparameter ermittelt (3). Die aktuellen Messwerte der messbaren Prozessparameter und die aktuellen ermittelten Werte der zusätzlichen Prozessparameter werden auf die jeweils zeitlich korrelierende Konzentration der Biomasse bezogen. Aus einer Kombination der aktuellen Konzentration der Biomasse und den aktuellen Messwerten der messbaren Prozessparameter sowie den ermittelten aktuellen Werten der zusätzlichen Prozessparameter werden dann aktuelle, zellspezifische metabolische Kennzahlen abgeleitet (4), welche dann in Verbindung mit einem deterministischen Prozessmodell verwendet werden (5). Dadurch wird eine biochemische Sicht auf biotechnologische Prozesse, insbesondere des Zellstoffwechsels, geboten. Die aktuellen, zellspezifischen metabolischen Kennzahlen können für eine bessere Regelung des biotechnologischen Prozesses herangezogen werden und um Fehler bei Messsensoren frühzeitig erkennen zu können.

## Beschreibung

### Technisches Gebiet

Die gegenständliche Erfindung betrifft allgemein das Gebiet der Biotechnologie und des so genannten Bio-Engineerings. Im Speziellen bezieht sich die vorliegende Erfindung auf ein Verfahren zur Kontrolle eines biotechnologischen Prozesses. Bei diesen biotechnologischen Prozessen werden während eines Prozessverlaufs Ausgangsstoffe mit Hilfe von Biomasse wie beispielsweise lebenden Zellen und/oder Mikroorganismen in Produkte umgesetzt. Dabei werden während des Prozessverlaufs wichtige Prozessparameter für eine Überwachung ermittelt.

### Stand der Technik

Heutzutage werden biotechnologische Prozesse in verschiedenen Bereichen wie z.B. in der Lebensmittel- und Getränkeindustrie, in der biopharmazeutischen Industrie, bei der Herstellung von Biokraftstoffen, etc. eingesetzt. Biotechnologische Prozesse wie insbesondere Fermentationsprozesse bilden beispielsweise eine Schlüsseltechnik bei der Erzeugung von Bier, Whisky oder Wein, bei der Erzeugung von Biokraftstoff oder bei der Herstellung von Impfstoffen oder Antibiotika dar. Bei biotechnologischen Prozessen wie einer Fermentation oder Fermentierung werden üblicherweise organische Ausgangsstoffe eines Substrats wie z.B. Zucker, Glukose, etc. mit Hilfe von Biomasse durch enzymatische Umwandlung in Produkte wie z.B. Alkohol, Säuren, Gase, etc. umgesetzt. Ein biotechnologischer Prozess bzw. eine Fermentation wird bewusst durch Zugabe der so genannten Biomasse zu einem Substrat ausgelöst. Bei biotechnologischen Prozessen werden beispielsweise lebende Zellen und/oder Mikroorganismen wie z.B. Bakterien, Pilze oder sonstige biologische Zellkulturen als Biomasse eingesetzt.

Die biotechnologischen Prozesse laufen üblicherweise in so genannten Bioreaktoren ab, in welchen die Umgebungs- und Reaktionsbedingungen für den jeweiligen biotechnologischen Prozess gesteuert und optimiert werden können. Für Fermentationsprozesse werden die Bioreaktoren auch als Fermenter bezeichnet. Um dem von der eingesetzten Biomasse zu produzierenden Stoff unter möglichst optimalen Bedingungen bzw. in der gewünschten Konzentration zu erhalten, werden im Bioreaktor entsprechende Umgebungs- und/oder Prozessparameter für den jeweiligen biotechnologischen Prozess wie z.B. pH-Wert, Temperatur, Sauerstoffzufuhr, Stickstoffzufuhr, Glukosegehalt oder Rühreinstellungen, etc. geregelt und gesteuert. Biotechnologische Prozesse sind allerdings biologisch komplex und sehr empfindlich. Daher ist eine laufende genaue Überwachung eines biotechnologischen Prozesses notwendig, um die entsprechenden Umgebungsbedingungen im Bioreaktor für einen konsistenten und optimalen Verlauf des Prozesses sicher zu stellen und damit die eingesetzte Biomasse in der Nährlösung wachsen und den gewünschten Stoff produzieren kann.

Für eine Optimierung biotechnologischer Prozesse ist weiterhin eine genaue Kenntnis der biochemischen Stoffwechselvorgänge notwendig, um höchste Ausbeuten bei geringstem Ressourcenverbrauch zu erzielen. Allerdings ist eine laufende genaue Überwachung und Kontrolle von biotechnologischen Prozessen und dabei insbesondere von biochemischen Stoffwechselvorgängen nicht einfach zu realisieren. Viele der dazu benötigten Prozessparameter und zellspezifischen Kennzahlen wie z.B. eine Konzentration von Metaboliten - Metabolite sind Ausgangsstoffe oder Produkte bzw. Zwischenprodukte aus enzymatischen Reaktionen, welche natürlicherweise in Zellen und/oder Mikroorganismen vorkommen - oder eine Konzentration des Produkts und somit auch Umsatzraten, etc. können nicht direkt gemessen werden.

Heutzutage werden biotechnologische Prozesse häufig aus einer Anlagensicht heraus betrieben und gesteuert. D.h. in heutigen Anlagen bzw. Bioreaktoren werden beispielsweise mittels einer so genannten Closed Loop Regelung Umweltbedingungen (z.B. Temperatur, pH-Wert, Sauerstoffgehalt, etc.) auf einen bestimmten Wert eingestellt und meist konstant gehalten. Eine für den jeweiligen Prozess notwendige Nahrungszugabe wird häufig durch eine festgelegte so genannte Feed-Strategie gesteuert. Weiterhin werden beispielsweise Prozessparameter wie z.B. Basenverbrauch, Sauerstoffverbrauch und Kohlendioxyd-Emission für die Regelung gemessen, und um abgeleitete Größen für ein Verhalten der Biomasse bzw. für ein Zellverhalten zu bekommen.

Bei einer derartigen Prozesssteuerung und -kontrolle werden allerdings die biochemischen Vorgänge nicht im Einzelnen überwacht und geregelt, sondern es wird üblicherweise nur eine Summe erfasst. Die genauen Einstellungen für die Umweltbedingungen - d.h. die genauen Einstellungen für z.B. Temperatur, pH-Wert, Sauerstoffgehalt, Strategie für die Nahrungszufuhr, etc. werden üblicherweise in einer Prozessentwicklung festgelegt. D.h. die Einstellungen werden für eine bestimmte Prozessführung beispielsweise mittels mathematischen Modellierungen des Prozesses ermittelt und optimiert, wobei der Prozess dann während der Produktion nur mehr anhand der vorgegebenen Einstellungen gesteuert wird. Der Prozess kann aber nicht mehr abgeändert werden.

Treten allerdings während des Prozessverlaufs nicht beeinflussbare Veränderungen in den Produktionsbedingungen wie z.B. einer Änderung in der Zusammensetzung von Produktionslösung, Nahrung oder Medium bzw. im Verhalten der eingesetzten Organismen auf, so kann dies zu Verlusten bei der Ausbeute führen und/oder der Prozess entfernt sich unter Umständen mit der Zeit immer weiter von einem in der Prozessentwicklung festgelegten Optimum. Für die Verluste und/oder Abweichungen können unterschiedliche Ursachen vorliegen wie z.B. Fehler im Prozess (z.B. Verunreinigungen, falsche Begasung, etc.), biologische Variabilität (z.B. Unterschiede in der Vorkultur der verwendeten Biomasse; Unterschiede im Zellstamm, etc.) oder Variabilität in der Prozessführung (z.B. Unterschiede bei den Rohstoffen, Schwankungen in der Substratzusammensetzung, Unterschiede bei der technischen Ausrüstung). Diese unterschiedlichen Ursachen sind oft nur schwer zu ermitteln und kaum zu korrigieren, da Daten und Kennwerte für zugrundeliegende biochemische und/oder biologische Vorgänge fehlen. Weiterhin sind beispielsweise Messfehler von Sensoren (z.B. Drift während der Produktion) oder Fehler in Regelungsmechanismen in manchen Fällen nur schwer zu erkennen.

Derzeit stehen allerdings wenn während des Verlaufs eines biotechnologischen Prozesses direkt keine bzw. kaum Informationen über zellspezifische metabolische Kennzahlen zur Verfügung, welche für eine korrigierende Regelung und Steuerung eines biotechnologischen Prozesses herangezogen werden könnten.

### Darstellung der Erfindung

Der Erfindung liegt daher die Aufgabe zugrunde, ein Verfahren zur Kontrolle eines biotechnologischen Prozesses anzugeben, bei welchem auf einfache und gesicherte Weise während eines Prozessverlaufs zellspezifische metabolische Kennzahlen ermittelt werden können, welche für eine Prozessentwicklung als auch für eine Prozesssteuerung herangezogen werden können.

Diese Aufgabe wird durch ein Verfahren der eingangs genannten Art mit den Merkmalen des unabhängigen Patentanspruchs gelöst. Vorteilhafte Ausführungsformen der vorliegenden Erfindung sind in den abhängigen Ansprüchen beschrieben.

Erfindungsgemäß erfolgt die Lösung der Aufgabe durch ein Verfahren der eingangs erwähnten Art, von welchem während des Prozessverlaufs eines biotechnologischen Prozesses periodisch wiederkehrend eine aktuelle Konzentration der im Prozess eingesetzten Biomasse abgeschätzt wird. Dann werden periodisch wiederkehrend aktuelle Messwerte von messbaren Prozessparametern bestimmt und daraus aktuelle Werte für zusätzliche Prozessparameter ermittelt. In der Folge werden die aktuellen Messwerte der messbaren Prozessparameter und die aktuellen ermittelten Werte der zusätzlichen Prozessparameter auf die jeweils zeitlich korrelierende Konzentration der Biomasse bezogen. D.h. in einem periodisch wiederkehrenden Messzyklus werden zuerst eine aktuelle Biomassekonzentration und dann entsprechend aktuelle Messwerte messbarerer Prozessparameter und daraus ermittelte aktuelle Werte von zusätzliche Prozessparameter bestimmt und dann miteinander kombiniert. Aus einer Kombination der aktuellen Konzentration der Biomasse und den aktuellen Messwerten der messbaren Prozessparameter und den ermittelten aktuellen Werten der zusätzlichen Prozessparameter werden dann aktuelle, zellspezifische metabolische Kennzahlen abgeleitet. Die aktuellen, zellspezifischen metabolischen Kennzahlen werden dann in Verbindung mit einem so genannten deterministischen Prozessmodell verwendet.

Der Hauptaspekt der erfindungsgemäß vorgeschlagenen Lösung besteht darin, dass durch die zur Verfügung gestellten aktuellen, zellspezifischen metabolischen Kennzahlen für quasi jeden Zeitpunkt im biotechnologischen Prozess direkte Informationen über zellbiologische Größen erhalten werden. Die zellspezifischen metabolischen Kennzahlen können dabei beispielsweise zellspezifische Aufnahmeraten von Sauerstoff und Nahrung (z.B. Glukose), zellspezifische Umsatz- oder Produktionsraten, spezifisches Zellwachstum, zellspezifische Kohlendioxyd-Emission, etc. umfassen. Es werden damit als aktuelle, zellspezifische metabolische Kennzahlen während des Prozessverlaufs gesicherte und aktuelle Werte für Zellparameter erhalten, welche eine biologische oder biochemische Sicht auf den jeweiligen biotechnologischen Prozess, insbesondere einen zugehörigen Zellstoffwechsel, ermöglichen. Die aktuellen, zellspezifischen metabolischen Kennzahlen können dann in Verbindung mit dem so genannten deterministischen Prozessmodell eingesetzt werden.

Mit Hilfe des so genannten deterministischen Prozessmodells können metabolische und katabolische Vorgänge während eines biotechnologischen Prozesses beschrieben werden. Dabei werden bekannte, biochemische Vorgänge innerhalb und außerhalb der jeweils eingesetzten bioaktiven Zellen (Biomasse) des jeweiligen biotechnologischen Prozesses mittels mathematischer Gleichungen wie z.B. Differentialgleichungen beschrieben, um beispielsweise Werte von wichtigen Prozessparametern (z.B. Glukosekonzentration, Ethanolkonzentration, Biomasse, etc.) zu jedem Zeitpunkt vorhersagen zu können. Dabei können als Anfangswerte für das deterministische Prozessmodell z.B. ein Anfangswert der Biomasse sowie ein Anfangswert einer für den jeweiligen biotechnologischen Prozess eingewogenen Nahrung (z.B. Glukose) herangezogen werden.

Das erfindungsgemäße Verfahren bietet weiterhin den Vorteil, dass Entscheidungen und Regelungen des Prozesses sehr einfach auf Basis von aktuellen, prozessspezifischen biologischen oder biochemischen Informationen durchgeführt werden können. Durch das erfindungsgemäße Verfahren sind beispielsweise die Konzentration der Biomasse, die Konzentrationen der Metaboliten sowie die Umsatz- oder Produktionsraten zu jedem Zeitpunkt eines biotechnologischen Prozesses bekannt und können gegebenenfalls auch direkt geregelt werden. Weiterhin kann auf Basis der aktuellen, zellspezifischen metabolischen Kennzahlen, insbesondere in Verbindung mit dem so genannten deterministischen Prozessmodell auf gegebenenfalls aufgetretene Messfehler oder Sensordefekt rückgeschlossen werden.

Es ist dabei günstig, wenn die aktuellen Werte der zusätzlichen Prozessparameter mittels eines so genannten bilanzierenden Prozessmodells aus den gemessenen aktuellen Werten der messbaren Prozessparameter berechnet werden. Durch das so genannte bilanzierende Prozessmodell wird aus Messwerten von messbaren Prozessparametern eine Stoffbilanz über den jeweiligen biotechnologischen Prozess hergestellt. Dazu werden chemische Reaktionsgleichungen verwendet, durch welche einzelnen Stoffwechselpfad für Metabolismus (= Stoffwechsel) und Anabolismus (= Zellwachstum) des jeweiligen Prozesses beschrieben werden. Das bilanzierende Prozessmodell weist dabei den Vorteil auf, dass ohne Angabe von Startbedingungen zeitaktuelle Werte für zusätzliche Prozessparameter ermittelt werden können. D.h. es können auch mit unbekannten oder variierenden Startbedingungen Werte für zusätzliche Prozessparameter aus den Messwerten der messbaren Prozessparameter sehr einfach bestimmt werden.

Als messbare Prozessparameter werden idealerweise solche Prozessparameter verwendet, für welche während des Verlaufs des biotechnologischen Prozesses annähernd in Echtzeit aktuelle Messwerte bestimmt werden können. Derartige messbare Prozessparameter sind beispielsweise ein Sauerstoffverbrauch und/oder eine Kohlendioxyd-Emissionen, deren aktuelle Messwerte sehr einfach mittels einer so genannten Off-Gas-Analyse bestimmt werden können. Die jeweils aktuellen Messwerte von Sauerstoffverbrauch und Kohlendioxyd-Emission stellen dann beispielsweise Eingangsparameter für das bilanzierende Prozessmodell dar, um aktuelle Werte zusätzlicher Prozessparameter zu ermitteln. Weitere messbare Prozessparameter, die ebenfalls im erfindungsgemäßen Verfahren eingesetzt werden können, sind beispielsweise ein Basenverbrauch oder ein Säureverbrauch je biotechnologischen Prozess.

Als zusätzliche Prozessparameter werden auf einfache Weise ableitbare Verbrauchs- und Produktionsgrößen verwendet. Derartige ableitbaren Verbrauchs- und Produktionsgrößen sind beispielsweise ein Nährstoffverbrauch, insbesondere ein Glukoseverbrauch, eine Stoffproduktion und/oder eine Biomassewachstum sowie aktuelle Stoffumsatzraten oder Produktionsraten. Die aktuellen Werte dieser zusätzlichen Produktionsparameter werden mit Hilfe des bilanzierenden Prozessmodells ermittelt.

Weiterhin ist es vorteilhaft, wenn für ein Abschätzen der jeweils aktuellen Konzentration der Biomasse eine geeignete Sensorik eingesetzt wird. Als geeignete Sensorik zum Abschätzen der Konzentration der Biomasse während des Prozessverlaufs kann z.B. ein System zum Messen einer Zelldichte von lebenden Zellen und/oder Mikroorganismen (z.B. Incyte von der Firma Hamilton) eingesetzt werden. Ein derartiges System basiert beispielweise darauf, dass sich lebende Zellen wie kleine Kapazitäten verhalten und ihre Polarisierung und Depolarisierung in einem veränderlichen elektrischen Feld gemessen werden kann. Aus dem gemessenen Signal kann während eines Prozessverlaufs die Zelldichte der Biomasse bzw. ein Bio-Volumen bestimmt und damit eine aktuelle Konzentration der Biomasse abgeschätzt werden.

Alternativ oder zusätzlich, kann für das Abschätzen der jeweils aktuellen Konzentration der Biomasse auch ein so genanntes statistisches Modell eingesetzt werden. Bei der mathematischen Modellierung eines statistischen Modells werden meist historische bzw. statische Daten des jeweiligen Bioprozesses (z.B. Werte von messbaren Prozessparametern wie z.B. Sauerstoffverbrauch, Basenverbrauch, Kohlendioxyd-Emission und Respiratorischer Quotient) herangezogen. Bei z.B. einer Annahme eines gleichbleibenden biotechnologischen Prozesses kann mit dem statischen Modell eine aktuelle Konzentration der Biomasse im Bioreaktor abgeschätzt bzw. für einen Messzeitpunkt vorhergesagt werden. Als statistische Modell kann beispielsweise ein so genanntes Partial-Least-Squares- oder PLS-Modell verwendet werden.

Eine zweckmäßige Weiterbildung des erfindungsgemäßen Verfahrens sieht vor, dass die aktuellen, zellspezifischen metabolischen Kennzahlen mit zeitlich korrelierenden, errechneten Vergleichswerten für diese zellspezifischen, metabolischen Kennzahlen verglichen werden. Für eine Berechnung der Vergleichswerte kann das deterministische Prozessmodell eingesetzt werden. Bei einer Differenz zwischen den aktuellen, zellspezifischen metabolischen Kennzahlen und den zeitlich korrelierenden errechneten Vergleichswerten können entsprechenden Korrektur- und/oder Überprüfungsmaßnahmen eingeleitet werden. Besonders vorteilhaft ist dabei, dass aus der Differenz zwischen den aktuellen, zellspezifischen metabolischen Kennzahlen und den zeitlich korrelierenden Vergleichswerten aus dem deterministischen Prozessmodell auf Messfehler bei eingesetzten Sensoren geschlossen werden kann.

Auf diese Weise können bereits kleine Fehler oder Schwankungen rasch entdeckt werden, da hier die aktuell ermittelten zellspezifischen, metabolischen Kennzahlen mit Vergleichswerten verglichen werden. Sind die Differenzen bzw. Abweichungen größer als zu erwartende statistische Schwankungen, so kann dies z.B. bereits als Fehler gewertet und entsprechend rasch in den Prozess eingegriffen werden.

Wird eine Differenz zwischen den aktuellen, zellspezifischen metabolischen Kennzahlen und den mit dem deterministischen Prozessmodell errechneten zeitlich korrelierenden Vergleichswerten z.B. größer als zu erwartende statistische Schwankungen festgestellt, so kann daraus geschlossen werden, dass sich die Biomasse beispielsweise anders als angenommen verhält. Es kann dann gegebenenfalls eine Nachregelung im Prozessverlauf des biotechnologischen Prozesses vorgenommen werden.

Weiterhin kann anhand der Differenzen oder Abweichungen zwischen den aktuellen, zellspezifischen metabolischen Kennzahlen und den mit dem deterministischen Prozessmodell errechneten zeitlich korrelierenden Vergleichswerten auf Messfehler von einzelnen Sensoren (z.B. Sauerstoffsonde, CO2-Sonde, etc.) geschlossen werden. Es kann dazu beispielsweise anhand der Wertekombination für den jeweiligen Zeitpunkt auf Eingangswerte zurückgerechnet und geprüft werden, ob eine derartige Eingangswertekombination chemisch und/oder physikalisch möglich ist (wie z.B. ein negativer Wert für die Biomasse, etc.).

Alternativ oder zusätzlich, kann es vorteilhaft sein, wenn die aktuellen, zellspezifischen metabolischen Kennzahlen als Anfangswerte oder Eingangswerte für ein deterministisches Prozessmodell herangezogen werden, wobei sich diese Werte aus den genannten statistischen und/oder bilanzierenden Prozessmodellen ergeben. In diesem Fall werden die jeweils aktuellen, zellspezifischen metabolischen Kennzahlen ins deterministische Prozessmodell übernommen, um beispielsweise einen weiteren Prozessverlauf, einen Zeitpunkt für ein Prozessende oder eine zu erwartende Prozessmenge vorherzusagen. Damit können - insbesondere in einer Prozessentwicklung - rasch und einfach Auswirkungen von geänderten Umweltbedingungen (z.B. Temperatur, pH-Wert, Sauerstoffangebot, etc.) abgeschätzt werden oder es kann ein deterministisches Prozessmodell sehr einfach für einen neuen Prozess (z.B. mit einer neuen Biomasse) parametriert werden.

Eine bevorzugte Ausführungsvariante des erfindungsgemäße Verfahrens sieht vor, dass eine zeitlich Frequenz für das Abschätzen der jeweils aktuellen Konzentration der Biomasse und die Bestimmung der aktuellen Messwerte der messbare Prozessparameter sowie die aktuellen Werte der zusätzlichen Prozessparameter - d.h. die zeitliche Wiederholungsfrequenz eines Messzyklus - in Abhängigkeit von der eingesetzten Biomasse festgelegt wird. D.h. die Wiederholfrequenz kann an den jeweiligen biotechnologischen Prozess bzw. seinen Prozessverlauf angepasst werden, um eine optimale Anzahl an aktuellen Werten für den jeweiligen Prozess zu bekommen.

Idealerweise werden die aktuellen, zellspezifischen metabolischen Kennzahlen auf einer Anzeigeeinheit ausgegeben. Dadurch stehen während des Prozessverlaufs einem Operator immer aktuelle Werte der zellspezifischen metabolischen Kennzahlen zur Verfügung, um beispielsweise den biotechnologischen Prozess überwachen, einzelne Prozessparameter direkt regeln oder Abweichungen sofort erkennen zu können.

### Kurzbeschreibung der Zeichnung

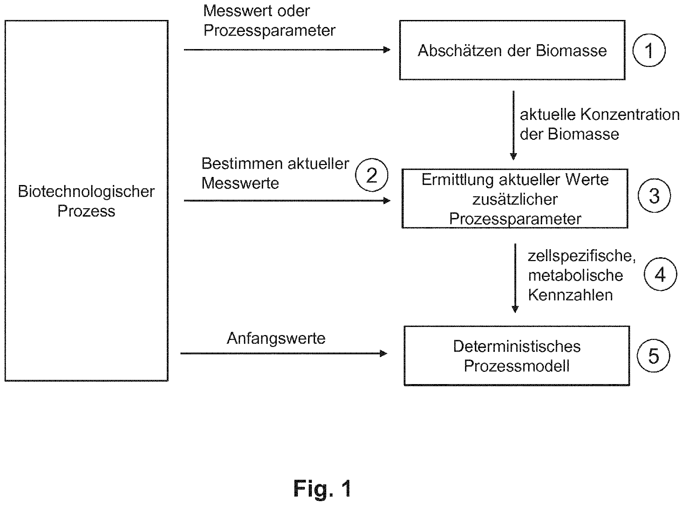

Die Erfindung wird nachfolgend in beispielhafter Weise anhand der beigefügten Figur 1 erläutert. Figur 1 zeigt schematisch einen beispielhaften Ablauf des erfindungsgemäßen Verfahrens zur Kontrolle eines biotechnologischen Prozesses.

### Ausführung der Erfindung

Figur 1 zeigt schematisch einen beispielshaften Ablauf des erfindungsgemäßen Verfahrens zur Kontrolle eines biotechnologischen Prozesses wie z.B. einer Batch-Fermentation, bei welcher als Biomasse Hefe eingesetzt wird. Bei einer Batch-Fermentation wird beispielsweise ein Reaktionsgefäß wie z.B. ein Fermenter mit den Ausgangsstoffen (z.B. Nahrung, etc.) und Biomasse befüllt. Bei dem beispielhaften biotechnologischen Prozess bzw. bei der beispielhaften Batch-Fermentation werden z.B. 61g Biomasse (Hefe) als Anfangsbiomasse und 56g Glukose als Nahrungsstartkonzentration verwendet. Die Anfangsbiomasse und die Nahrungsstartkonzentration stellen auch Anfangswerte bzw. Eingangsgrößen für ein deterministisches Prozessmodell dar, mit welchen metabolische und katabolische Vorgänge während des biotechnologischen Prozesses beschrieben werden können. Mit Hilfe des deterministischen Prozessmodells kann der Prozessverlauf des biotechnologischen Prozesses bzw. der Hefe-Batch-Fermentation abgeschätzt werden. Aus dem deterministischen Prozessmodell lassen sich weiterhin Vergleichswerte für zellspezifische metabolische Kennzahlen wie z.B. Glukosekonzentration, Ethanolkonzentration, Biomasse, etc. zu jedem Zeitpunkt während des Prozessverlaufs bestimmen.

Während des beispielhaften biotechnologischen Prozesses - der Hefe-Batch-Fermentation - erfolgt nach Befüllung des Fermenters eine Reaktion zwischen Biomasse und Ausgangsstoffen (z.B. Glukose, etc.), deren Konzentration kontinuierlich fällt, zu Produkten, der Konzentration kontinuierlich ansteigt. Bei der Hefe-Batch-Fermentation wird beispielsweise in einer ersten Phase die Nahrung bzw. die Glukose von der Biomasse bzw. der Hefe verbraucht. Dabei wird ein Teil der Glukose mit Sauerstoff zu Kohlendioxyd oxidiert und ein Teil der Glukose zu Ethanol fermentiert. In einer zweiten Phase stellen sich die Hefezellen beispielsweise auf Ethanolverbrauch um, wobei in dieser Phase der Metabolismus der Hefezellen stark zurückgeht. In einer dritten Phase der Hefe-Batch-Fermentation wird dann das Ethanol verbraucht.

Während des biotechnologischen Prozesses können weiterhin wichtige messbare Prozessparameter wie z.B. Sauerstoffverbrauch, Kohlendioxyd-Emission und der daraus abgeleitete, so genannte respiratorische Quotient (RQ) beobachtet werden. Der Sauerstoffverbrauch sowie die Kohlendioxyd-Emission werden dazu beispielsweise mittels einer so genannten Off-Gas-Analyse bestimmt. Aufgrund der metabolischen Vorgänge während des biotechnologischen Prozesses bzw. während der Hefe-Batch-Fermentation wird das Fermentermedium in den unterschiedlichen Phasen unterschiedlich stark angesäuert. Dem wird mittels Basenzugabe entgegen gewirkt, um eine pH-Wert im Fermenter konstant zu halten. Daher kann bei der Hefe-Batch-Fermentation auch ein Basenverbrauch als messbarer Prozessparameter genutzt bzw. beobachtet werden. Bei anderen biotechnologischen Prozessen kann beispielsweise auch ein Säureverbrauch als messbarer Prozessparameter herangezogen werden. Während des gesamten Prozessverlaufs der beispielshaften Hefe-Batch-Fermentation wachsen die als Biomasse eingesetzten Hefezellen. Das bedeutet auch die Biomasse verändert sich während des Prozessverlaufs - bei der Hefe-Batch-Fermentation nimmt die Biomasse z.B. zu.

Daher wird während des Prozessverlaufs der beispielhaften Hefe-Batch-Fermentation in einem ersten Verfahrensschritt 1 eine aktuelle Konzentration der Biomasse im Fermenter eine abgeschätzt. Ein Abschätzen der aktuellen Konzentration der Biomasse kann dabei beispielsweise mit Hilfe einer geeigneten Sensorik wie z.B. mit einem System zum Messen einer Zelldichte von lebenden Zellen und/oder Mikroorganismen (z.B. Incyte von der Firma Hamilton) erfolgen. Alternativ oder gegebenenfalls zusätzlich, kann die aktuelle Konzentration der Biomasse im Fermenter auch mittels eines so genannten statistischen Modells aus messbaren Prozessparametern abgeschätzt werden. Dazu wird z.B. ein so genanntes Partial-Least-Squares- oder PLS-Modell verwendet, mit dessen Hilfe aus aktuellen Werten von beispielsweise Sauerstoffverbrauch, Kohlendioxyd-Emission, Respiratorischem Quotienten und Basenverbrauch die Konzentration der Biomasse abgeschätzt wird.

In einem zweiten Verfahrensschritt 2 werden aktuelle Messwerte von messbaren Prozessparametern bestimmt. Als messbare Prozessparameter können dabei alle jene Prozessparameter verwendet werden, für welche leicht und rasch (d.h. annähernd in Echtzeit) aktuelle Messwerte während des Prozessverlaufs bestimmbar sind. Für die Hefe-Batch-Fermentation können während des Prozessverlaufs z.B. sehr einfach der Sauerstoffverbrauch und/oder die Kohlendioxyd-Emission gemessen werden. Die Messung erfolgt dabei beispielsweise mittels der so genannten Off-Gas-Analyse. Weiterhin kann ein Basenverbrauch sehr einfach zu jedem Zeitpunkt während des Prozessverlaufs bestimmt werden, indem z.B. ein Gewicht der noch nicht in den Fermenter zugegebenen Base gemessen wird.

Aus den aktuellen Messwerten der messbaren Prozessparameter werden dann in einem dritten Verfahrensschritt 3 zugehörige, aktuelle Werte für zusätzliche Prozessparameter ermittelt. Dabei werden als zusätzliche Prozessparameter abgeleitete Verbrauchs- und Produktionsgrößen, insbesondere ein Nährstoff- oder Glukoseverbrauch, eine Stoff- oder Ethanolproduktion und/oder eine Biomassewachstum. Weiterhin können auch aktuelle Werte für Stoffumsatzraten und/oder Produktionsraten ermittelt werden. Die aktuellen Werte der zusätzlichen Produktionsparameter werden im dritten Verfahrensschritt 3 mit Hilfe eines so genannten bilanzierenden Prozessmodells bestimmt.

Vom bilanzierenden Prozessmodell werden beispielsweise den jeweiligen biotechnologischen Prozess - z.B. die Hefe-Batch-Fermentation - beschreibende chemische Reaktionsgleichungen verwendet. Durch diese chemischen Reaktionsgleichungen werden die einzelnen Stoffwechselpfad für Metabolismus (= Stoffwechsel) und Anabolismus (= Zellwachstum) des jeweiligen Prozesses beschrieben. Bei der Hefe-Batch-Fermentation werden für das bilanzierende Prozessmodell beispielsweise Stoffwechselpfade verwendet, welche Glukose-Oxidation und Glukose-Fermentation in der ersten Prozessphase sowie einen EthanolVerbrauch in der zweiten und vor allem in der dritten Prozessphase beschreiben. Dabei werden die aktuellen Messwerte der leicht messbaren Prozessparameter "Sauerstoffverbrauch" und "Kohlendioxyd-Emission" als Eingangswerte für das bilanzierende Prozessmodell genutzt und unter einer Berücksichtigung einer Stickstoff-Bilanz zur Abschätzung des Biowachstums, aus dem bilanzierenden Modell die aktuellen Werte einer Glukose- und Ethanolkonzentration als zusätzliche Prozessparameter für die Hefe-Batch-Fermentation bestimmt.

Dann werden die aktuellen Messwerte der messbaren Prozessparameter sowie die entsprechenden aktuellen Werte der zusätzlichen Prozessparameter, welche aus den aktuellen Messwerten der messbaren Prozessparameter bestimmt wurden, auf die entsprechende aktuelle (d.h. zeitlich korrelierende) Konzentration der Biomasse bezogen. In einem vierten Verfahrensschritt 4 werden dann daraus aktuelle zellspezifische metabolische Kennzahlen abgeleitet. Diese aktuellen zellspezifischen metabolischen Kennzahlen können beispielsweise für die Hefe-Batch-Fermentation zellspezifische Aufnahmeraten von Sauerstoff und Glukose, zellspezifische Umsatz- oder Produktionsraten wie z.B. zellspezifische Raten an oxidierte Glukose und/oder zu Ethanol fermentierter Glukose, spezifisches Zellwachstum, zellspezifische Kohlendioxyd-Emission, etc. umfassen.

Weiterhin können die aktuellen Werte der zellspezifischen metabolischen Kennzahlen und/oder eine jeweilige zeitliche Veränderung der zellspezifischen metabolischen Kennzahlen auf einer Anzeigeeinheit ausgegeben werden. Ein Operator erhält dadurch ein umfassendes Bild über die biologischen und biochemischen Vorgänge während des Prozesses und kann Entscheidungen und Regelungen direkt auf Basis der erhaltenen biologischen Information bzw. der aktuellen Werte der zellspezifischen metabolischen Kennzahlen durchführen.

Weiterhin können die jeweils aktuellen, zellspezifischen metabolischen Kennzahlen in einem fünften Verfahrensschritt 5 mit entsprechenden, zeitlich korrelierenden Vergleichswerten verglichen werden. Diese Vergleichswerte werden beispielsweise mit Hilfe des deterministischen Prozessmodells berechnet, mit welchem beispielsweise während einer Prozessentwicklung metabolische und katabolische Vorgänge während des biotechnologischen Prozesses beschrieben werden können. Als Anfangswerte für das deterministische Prozessmodell wurden bei der beispielhaft beschriebenen Hefe-Batch-Fermentation - wie bereits ausgeführt - die eingewogene Anfangsbiomasse (Hefe) und die Nahrungsstartkonzentration (Glukosemenge) verwendet. Werden bei dem Vergleich Differenzen bzw. Abweichungen zwischen einer oder mehreren aktuellen, zellspezifischen metabolischen Kennzahlen und der bzw. den entsprechenden Vergleichswerten festgestellt, welche größer als zu erwartende statische Schwankungen sind, so können entsprechende Korrektur- und/oder Überprüfungsmaßnahmen eingeleitet werden. Es kann beispielsweise - wenn aufgrund der Prozessführung keine Änderungen von zellspezifische metabolischen Kennzahlen (z.B. der Umsatzraten) zu erwarten sind oder wenn die Abweichungen nicht durch Änderungen von zellspezifischen metabolischen Kennzahlen erklärt werden können - aus den festgestellten Abweichungen auf Messfehlern der Sensoren (z.B. Sauerstoffsonde, CO2-Sonde, etc.) geschlossen werden. Weiterhin können die Abweichungen dazu genutzt werden, um gegebenenfalls regelnd in den Prozess einzugreifen.

Da mit dem erfindungsgemäßen Verfahren die aktuelle Konzentration der Biomasse, die aktuellen Messwerte der messbaren Prozessparameter und die aktuellen Werte der zusätzlichen Prozessparameter und damit auch die aktuellen, zellspezifischen metabolischen Kennzahlen periodisch wiederkehrend bestimmt werden, können im fünften Verfahrensschritt 5 bereits kleine Fehler rasch entdeckt werden.

Alternativ können die im vierten Verfahrensschritt 4 abgeleiteten, zellspezifischen metabolischen Kennzahlenwerte auch in eine Prozessvorhersage bei variierender Prozessführung verwendet werden. Dazu werden aktuell ermittelte Werte der zellspezifischen metabolischen Kennzahlen vom deterministischen Prozessmodell im fünften Verfahrensschritt 5 quasi als Anfangswerte übernommen, um einen weiteren Prozessverlauf wie z.B. eine Zeitpunkt des Prozessendes oder eine zu erwartende Produktmenge vorherzusagen. Auf diese Weise können beispielsweise Auswirkungen von sich ändernden Umweltbedingungen (z.B. Temperatur, pH-Werte, Medium oder Sauerstoffangebot) auf die Umsatzraten abgeschätzt werden. Weiterhin kann auf diese Weise das deterministische Modell für einen neuen biotechnologischen Prozess - z.B. mit einem neuen Hefestamm, mit einer anderen Biomasse, etc. - rasch adaptiert und parametriert werden.

Bei dem erfindungsgemäßen Verfahren werden die aktuelle Konzentration der Biomasse, die aktuellen Messwerte der messbaren Prozessparameter und die aktuellen Werte der zusätzlichen Prozessparameter und damit auch die aktuellen, zellspezifischen metabolischen Kennzahlen mit einer zeitlichen Frequenz periodisch wiederkehrend bestimmt. Das bedeutet, dass zumindest die ersten vier Verfahrensschritte 1 bis 4 periodisch wiederkehrend mit einer Wiederholfrequenz durchgeführt werden. Diese ersten vier Verfahrensschritte 1 bis 4 können beispielsweise zu einem Messzyklus zusammengefasst werden, welcher mit der Wiederholfrequenz bzw. mit der zeitlichen Frequenz wiederholt wird. Diese zeitliche Frequenz bzw. Wiederholfrequenz kann in Abhängigkeit von der eingesetzten Biomasse festgelegt werden. Bei der Hefe-Batch-Fermentation kann die Wiederholfrequenz beispielsweise mit einmal pro Minute festgelegt werden.

Für eine beispielhafte praktische Anwendung des erfindungsgemäßen Verfahrens kann der Fermenter beispielsweise mit einem Prozessleitsystem wie z.B. der Simatic PCS7 gesteuert werden. Für den Ablauf des erfindungsgemäßen Verfahrens werden z.B. mit der festgelegten Wiederholfrequenz (z.B. einmal pro Minute) die aktuellen Werte der messbaren Prozessparameter und/oder die Off-Gas-Analysewerte über einen Schnittstelle an eine so genanntes Process Analytical Technology (PAT) System wie z.B. Simatic SIPAT weitergegeben. Ein Process Analytical Technology System dient einer Optimierung, Analyse und Kontrolle von Herstellungsprozessen in der chemischen und biotechnologischen Industrie.

Durch das Process Analytical Technology System wird dann mit der Wiederholfrequenz wie z.B. einmal in der Minute zuerst der erste Verfahrensschritt 1 getriggert, um eine entweder mittels geeigneter Sensorik oder mit Hilfe des statistischen Modells die gerade aktuelle Konzentration der Biomasse abzuschätzen. Anschließend wird durch das Process Analytical Technology System der zweite und dritte Verfahrensschritt 2, 3 angestoßen, um aktuelle Messwerte der messbaren Prozessparameter und aktuelle Werte der zusätzlichen Prozessparameter aus dem bilanzierenden Prozessmodell zu erhalten. Im vierten Verfahrensschritt 4 werden dann die aktuellen, zellspezifischen metabolischen Kennzahlen abgeleitet, welche dann mit den durch das deterministische Prozessmodell berechneten Vergleichswerten im fünften Verfahrensschritt 5 für eine Prozesskontrolle und Fehleranalyse verglichen werden. Alternativ kann im fünften Verfahrensschritt 5 ein weiterer Prozessverlauf mit dem deterministische Prozessmodell und den aktuellen, zellspezifischen metabolischen Kennzahlen als Anfangswerte berechnet werden.

Nach dem Durchlauf der Verfahrensschritt 1 bis 4 bzw. der Verfahrensschritt 1 bis 5 wird der Messzyklus wieder gestartet und mit neuen, aktuellen Messwerten von messbaren Prozessparametern (z.B. Biomasse, Sauerstoffverbrauch, Kohlendioxyd-Emission) durchlaufen.

Das erfindungsgemäße Verfahren wurde beispielhaft anhand einer Hefe-Batch-Fermentation bzw. anhand eines Batch-Verfahrens beschrieben. Es ist aber auch für andere biotechnologische Prozesse einsetzbar, bei welchen beispielsweise das so genannte Fed-Batch-Verfahren verwendet wird.

## Patentansprüche

1. Verfahren zur Kontrolle eines biotechnologischen Prozesses, bei welchem während eines Prozessverlaufs Ausgangsstoffe mit Hilfe von Biomasse, insbesondere lebenden Zellen und/oder Mikroorganismen, in Produkte umgesetzt werden, und wobei wichtige Prozessparameter ermittelt werden, ***dadurch gekennzeichnet, dass*** während des Prozessverlaufs des biotechnologischen Prozesses periodisch wiederkehrend eine aktuelle Konzentration der im Prozess eingesetzten Biomasse abgeschätzt wird (1),
dass periodisch wiederkehrend aktuelle Messwerte von messbaren Prozessparametern bestimmt werden (2),
dass aus den aktuellen Messwerten der messbaren Prozessparametern aktuelle Werte für zusätzliche Prozessparameter ermittelt werden (3),
dass die aktuellen Messwerte der messbaren Prozessparametern und aktuellen ermittelten Werte der zusätzlichen Prozessparameter auf die jeweils zeitlich korrelierende Konzentration der Biomasse bezogen werden,
dass daraus aktuelle, zellspezifische metabolische Kennzahlen abgeleitet werden (4) und dass die aktuellen, zellspezifischen metabolischen Kennzahlen in Verbindung mit einem so genannten deterministischen Prozessmodell verwendet werden (5).

2. Verfahren nach Anspruch 1, ***dadurch gekennzeichnet, dass*** die aktuellen Werte der zusätzlichen Prozessparameter mittels eines so genannten bilanzierenden Prozessmodells aus den gemessenen aktuellen Werten der messbaren Prozessparameter berechnet werden (3).

3. Verfahren nach einem der Ansprüche 1 bis 2, ***dadurch gekennzeichnet, dass*** als messbare Prozessparameter solche Prozessparameter verwendet werden, für welche während des Verlaufs des biotechnologischen Prozesses annähernd in Echtzeit aktuelle Messwerte bestimmt werden können (2).

4. Verfahren nach einem der Ansprüche 1 bis 3, ***dadurch gekennzeichnet, dass*** als zusätzliche Prozessparameter ableitbare Verbrauchs- und Produktionsgrößen, verwendet werden (3).

5. Verfahren nach einem der Ansprüche 1 bis 4, ***dadurch gekennzeichnet, dass*** für ein Abschätzen der jeweils aktuellen Konzentration der Biomasse eine geeignete Sensorik eingesetzt wird (1).

6. Verfahren nach einem der Ansprüche 1 bis 4, ***dadurch gekennzeichnet, dass*** für ein Abschätzen der jeweils aktuellen Konzentration der Biomasse ein so genanntes statistisches Modell eingesetzt wird (1).

7. Verfahren nach einem der Ansprüche 1 bis 6, ***dadurch gekennzeichnet, dass*** die aktuellen, zellspezifischen metabolischen Kennzahlen mit zeitlich korrelierenden, errechneten Vergleichswerten für diese zellspezifischen metabolischen Kennzahlen verglichen werden, wobei für eine Berechnung der Vergleichswerte das deterministische Prozessmodell eingesetzt wird (5), und dass bei einer Differenz zwischen den aktuellen, zellspezifischen metabolischen Kennzahlen und den zeitlich korrelierenden errechneten Vergleichswerten Korrektur- und/oder Überprüfungsmaßnahmen eingeleitet werden.

8. Verfahren nach Anspruch 7, ***dadurch gekennzeichnet, dass*** aus der Differenz zwischen den aktuellen, zellspezifischen metabolischen Kennzahlen und den zeitlich korrelierenden Vergleichswerten aus dem deterministischen Prozessmodell auf Messfehler bei eingesetzten Sensoren geschlossen werden kann.

9. Verfahren nach einem der Ansprüche 1 bis 6, ***dadurch gekennzeichnet, dass*** die aktuellen, zellspezifischen metabolischen Kennzahlen als Anfangswerte für das deterministische Prozessmodell herangezogen werden (5).

10. Verfahren nach einem der vorangegangenen Ansprüche, ***dadurch gekennzeichnet, dass*** eine zeitliche Frequenz für das Abschätzen der jeweils aktuellen Konzentration der Biomasse und die Bestimmung der aktuelle Messwerte der messbaren Prozessparameter und der aktuellen Werte der zusätzlichen Prozessparameter in Abhängigkeit von der eingesetzten Biomasse festgelegt wird.

11. Verfahren nach einem der vorangegangenen Ansprüche, ***dadurch gekennzeichnet, dass*** die aktuellen, zellspezifischen metabolischen Kennzahlen auf einer Anzeigeeinheit ausgegeben werden (4).
